# EUROPÄISCHE PATENTSCHRIFT

(11) **EP 2 622 067 B1**
(45) Veröffentlichungstag und Bekanntmachung des Hinweises auf die Patenterteilung: **14.05.2014**
(21) Anmeldenummer: 11751818.3
(22) Anmeldetag: 16.08.2011
(51) Int. Cl.: C12N 7/02

(54) **VERFAHREN ZUR ABTRENNUNG VON VIREN AUS EINEM KONTAMINANTEN ENTHALTENDEN FLÜSSIGEN MEDIUM**
METHOD FOR THE PURIFICATION OF VIRUSES FROM LIQUIDS CONTAINING CONTAMINANTS
PROCEDE POUR PURIFIER DES VIRUS DES LIQUIDS CONTENANT DES CONTAMINANTS

(30) Priorität: 28.09.2010 DE 102010046817
(43) Veröffentlichungstag der Anmeldung: 07.08.2013
(73) Patentinhaber: Sartorius Stedim Biotech GmbH, 37079 Göttingen (DE)
(72) Erfinder: FABER, René, 37077 Göttingen (DE); LEUTHOLD, Martin, 37079 Göttingen (DE)
(86) Internationale Anmeldenummer: PCT/EP2011/004109
(87) Internationale Veröffentlichungsnummer: WO 2012/041423

(56) Entgegenhaltungen:
- EP-A1- 1 878 791
- WO-A2-03/078592

## Beschreibung

Die vorliegende Erfindung betrifft ein Verfahren zur Abtrennung von Viren aus einem Kontaminanten enthaltenden flüssigen Medium, wobei die Viren zunächst an einem ersten Adsorbens mit kationischen Gruppen adsorbiert und nachfolgend von diesem desorbiert werden und wobei anschließend ein flüssiges Medium, welches die Viren und Kontaminanten enthält, zur Adsorption der Kontaminanten mit einem zweiten Adsorbens mit kationischen Gruppen kontaktiert wird.

Effizienten Verfahren zur Abtrennung von Viren aus biotechnologischen Flüssigkeiten, welche häufig unerwünschte Kontaminanten enthalten, kommt in der Medizin und Biotechnologie eine stetig wachsende Bedeutung zu.

Viren, Virionen oder virale Partikel bestehen aus einer Nukleinsäure (Desoxyribonukleinsäure (DNS) oder Ribonukleinsäure (RNS)) und aus einer Proteinhülle, auch Kapsid genannt. Einige Viren sind zusätzlich von einer Membran umgeben, die als Virushülle bezeichnet wird, oder besitzen andere zusätzliche Bestandteile. Solche Viren, die zusätzlich zum Kapsid eine Virushülle aufweisen, werden behüllt genannt, Viren ohne derartige Hülle bezeichnet man als unbehüllt. Viroide besitzen weder ein Kapsid noch eine Virushülle. Ein Virus selbst ist nicht zu Stoffwechselvorgängen fähig, daher braucht es Wirtszellen zur Fortpflanzung. Viren befallen Zellen von Eukaryonten (Pflanzen, Pilze, Tiere) und Prokaryonten (Bakterien und Archaeen). Viren, die Prokaryonten als Wirte nutzen, werden Bakteriophagen genannt.

Das Influenza-Virus ist ein umhülltes Viruspartikel mit einem Durchmesser von 80 bis 120 nm, in dessen Hülle eine jeweils unterschiedliche Anzahl an Proteinen und Glykoproteinen eingelagert ist. Das Genom fast aller Influenzaviren besteht aus acht RNS-Abschnitten negativer Polarität. Die Hauptproteine der Hülle sind das Hämagglutinin (HA) und die Neuraminidase (NA). Durch Virusmutationen besonders im Hinblick auf mögliche Veränderungen des Hämagglutinins kann sich die Infektionsgefahr für den potentiellen Wirt erheblich vergrößern.

Virusartige Partikel ("Virus-like particles", VLP) sind Viruspartikel, die biotechnologisch hergestellt werden. Sie enthalten keine viralen Nukleinsäuren und sind daher nicht in der Lage, sich in den Zielzellen zu vermehren. VLP werden in der Virologie und in der Immunologie benötigt, um Viren und Zellfunktionen zu untersuchen. Diese Partikel sind nicht leer, da sie sonst instabil würden, sondern es werden entweder unspezifisch Nukleinsäuren verpackt oder nicht funktionelle DNS bzw. RNS. Außerdem können gezielt Proteine verpackt werden.

Natürliche und/oder rekombinante Viren können in der Medizin als Impfstoffe (Vakzine) eingesetzt werden. Impfstoff sind präventive oder therapeutische Mittel, die ihren Effekt durch Stimulierung des Immunsystems eines Individuums erreichen. Attenuierte Lebendimpfstoffe enthalten abgeschwächte Viren, die sich noch vermehren und eine Immunantwort auslösen können, d. h. immunogen sind, in der Regel jedoch keine Erkrankung hervorrufen, d. h. nicht pathogen sind. Totimpfstoffe enthalten inaktivierte oder abgetötete Viren oder Bestandteile von Viren.

Eine weitere Art, Viren in der Medizin einzusetzen, findet sich in der Gentherapie. Mit Gentherapie bezeichnet man das Einfügen von Genen in Zellen eines Individuums zur Behandlung von Erbkrankheiten bzw. Gendefekten. Durch die Einführung dieser Gene kann ein genetischer Defekt kompensiert werden. Ein genetischer Defekt liegt vor, wenn bei einem Lebewesen ein Gen fehlt oder eine Mutation vorliegt, die dazu führt, daß das Genprodukt (z.B. ein Protein) nicht gebildet wird oder seine Funktion nicht richtig ausüben kann. Bei einer Gentherapie werden dem Körper Zellen entnommen. Diese Zellen erhalten das neue (therapeutische) Gen und werden anschließend wieder in den Körper eingebracht (ex vivo). Durch den Einsatz von viralen Vektoren kann eine Gentherapie direkt im Körper erfolgen (in vivo). Hier werden meist Retroviren oder Adenoviren benutzt, um DNS-Abschnitte in die Körperzellen des Patienten zu transferieren.

Die Herstellung von Viren, die entweder als Impfstoffe oder als Vektoren in der Gentherapie eingesetzt werden, ist von stetig zunehmender Relevanz für die Biotechnologie. Die Produktion erfolgt in mehreren Schritten:
1. Generierung eines Pathogens/Antigens,
2. Herstellung des Antigen in einem geeigneten System (z.B. Zellkultur, Hühnerembryonen),
3. Aufreinigung des Antigens und
4. Formulierung des Impfstoffs bzw. des Vektors durch Zusatz von Hilfsmitteln, Wirkverstärkern, Stabilisatoren, Konservierungsmitteln etc.

Nach der Kultivierung von Viren in Zellkulturen (z. B. MRC-5, vero, PER.C6) oder in Hühnereiern ist es notwendig, die Viren von den Kontaminanten (z. B. Wirtszellproteinen, DNS oder Endotoxinen) zu trennen, um sie in reiner Form für die gewünschte Applikation zu erhalten. Weiterhin ist es von Vorteil, infektiöse von nichtinfektiösen Molekülen bzw. Partikeln zu trennen. Viruseigenschaften, wie der isoelektrische Punkt (pI), Oberflächenhydrophobizität, Vorhandensein einer Hülle und der hydrodynamische Durchmesser, können zur Aufreinigung genutzt werden. Aufreinigungsmethoden basierend auf der Größe der Viren sind im Stand der Technik bekannt. Dazu zählen z.B.
- die Dichtegradient-Ultrazentrifugation (im CsCl- oder Sucrose-Gradient, nachteilig sind die hohen Investitionskosten),
- die Ultra- und Mikro-Filtration mit Flach- oder Hohlfasermembranen,
- die Fällung (z.B. mit Polyethylenglycol oder Ammoniumsulfat) oder
- die Größenausschlußchromatographie mit Chromatographiegelen, z.B. auf Agarosebasis. Nachteilig beim zuletzt genannten Verfahren sind niedrige Flussraten und die niedrige Prozeßgeschwindigkeit.

Zum Abbau von Wirtszell-DNS wird oft ein Enzym, wie z.B. die Endonuclease Benzonase®, verwendet. Der Nachteil dieser Methode sind sehr hohe Enzymkosten. Tiefenfilter werden zur Entfernung von Zellen und/oder Zellbruchstücken bei der Aufreinigung von Viren eingesetzt.

Die Adsorption von Viren an festen Phasen durch chromatographische Aufreinigung hat eine große Bedeutung in der Virusaufreinigung, insbesondere im Prozeßmaßstab. Adsorbentien sind poröse Feststoffe, die über als Liganden bezeichnete funktionelle Oberflächengruppen mit bestimmten Komponenten von Fluiden selektiv Bindungen eingehen können. Zielsubstanz(en) und/oder Kontaminant(en) werden als Adsorbenden bezeichnet, wobei es sich auch um mehrere unterschiedliche Substanzen handeln kann. Adsorbenden können Einzelmoleküle, Assoziate oder Partikel sein, bei denen es sich vorzugsweise um Viren, Virenbestandteile, virenartige Partikel, Proteine oder andere Substanzen biologischen Ursprungs handelt.

Die Bindung der Adsorbenden an das Adsorbens kann reversibel oder irreversibel sein, in jedem Fall ermöglicht sie ihre Abtrennung von den Fluiden, bei denen es sich im allgemeinen um wässrige Flüssigkeiten handelt und die im folgenden Medien genannt werden. Unter dem Begriff "Elution" werden die Desorption eines Adsorbenden von dem Adsorbens und die damit einhergehenden Spülschritte etc. zusammengefasst. Die zur Elution verwendete Flüssigkeit ist das Elutionsmittel. Die Komponenten können eine oder mehrere Zielsubstanzen und/oder eine oder mehrere Kontaminanten darstellen. Zielsubstanzen sind Wertstoffe, die aus dem Medium in angereicherter oder reiner Form gewonnen werden sollen. Zielsubstanzen können beispielsweise Viren sein. Kontaminanten sind Stoffe, deren Abwesenheit oder Entfernung aus dem Fluid aus technischen, regulatorischen oder sonstigen Gründen erforderlich oder wünschenswert ist. Kontaminanten können beispielsweise Wirtszellproteine, Aminosäuren, Nukleinsäuren, Endotoxine, Proteinaggregate, Liganden oder deren Teile sein. Für die Entfernung von Kontaminanten, die als "negative Adsorption" bezeichnet wird (auch "Flow-through", FT genannt), kann/darf die Adsorption irreversibel verlaufen, wenn das Adsorbens nur einmal verwendet werden soll. Bei der Adsorption der Zielsubstanz(en) muss der Vorgang reversibel verlaufen (auch "Bind-and-elute", B&E genannt). Es kann entweder eine bloße Anreicherung oder eine Auftrennung in mehrere Zielsubstanzen durchgeführt werden, wobei im letzteren Fall entweder die Adsorption, die Desorption oder beide selektiv erfolgen können.

Herkömmliche Adsorbentien für die Chromatographie sind partikulär geformt und werden in Form von Schüttungen in Säulen betrieben. Da Viren typischerweise eine Größe bis zu 1000 nm aufweisen, sind konventionelle Chromatographiegele mit Porengrößen im Bereich von 30 - 400 nm für die Virusaufreinigung meist ungeeignet. Die Viren können nur auf der äußeren Oberfläche der Partikel binden, woraus nur geringe Bindungskapazitäten resultieren. Verschiedene Liganden wurden bereits in der Virusaufreinigung verwendet: Anionenaustauscher ("anion exchange", AEX), Kationenaustauscher ("cation exchange", CEX), Affinitätsliganden ("affinity ligands", AF), Liganden für die hydrophobe Interaktionschromatographie (HIC) oder komplexbildende Liganden für die Metallchelatchromatographie ("Immobilized Metal Ion Affinity Chromatography", IMAC).

Im Stand der Technik sind zahlreiche Verfahren zur Abtrennung von Viren aus biotechnologischen Fluiden mittels verschiedener Chromatographiematrizen mit ionenaustauschenden Liganden beschrieben.

WO 03/078592 A2 beschreibt ein Verfahren zur Aufreinigung von Adenoviren, welche aus Zelllysaten gewonnen werden, mit Hilfe von zwei Anionenaustauscher-Filtern. Das Adenovirus wird dabei zunächst auf einem ersten Anionenaustauch-Filter reversibel im "Bind-and-Elute"-Modus gebunden. Danach wird das gewonnene Eluat nach einer Nuklease-Behandlung zum Abbau von Nukleinsäure-Kontaminanten auf einem zweiten Anionenaustausch-Filter reversibel im "Bind-and-Elute"-Modus gebunden.

US 6,261,823 B1 beschreibt eine Methode zur Aufreinigung von Adenoviren mit Hilfe eines ersten Anionenaustauscher-Chromatographieschritts mit DEAE-Fractogel gefolgt von einem zweiten Größenausschluß-Chromatographieschritt mit dem Gel Superdex-200. Das Virus wird im ersten Schritt reversibel im "Bind-and-Elute"-Modus auf dem Anionenaustauscher adsorbiert und anschließend eluiert.

US 5,837,520 beschreibt eine Methode zur Aufreinigung von viralen Vektoren, welche aus Zelllysaten nach Behandlung mit Nukleinsäure spaltenden Enzymen gewonnen worden sind. In einem ersten Schritt werden die viralen Partikel mit Hilfe eines ersten kationen- bzw. anionenaustauschenden Chromatographiegels und in einem zweiten Schritt mit einem Affinitätschromatographiegel behandelt, auf welchem zur Chelatbildung befähigte Metallionen immobilisiert sind. Alternativ kann im zweiten Schritt ein Chromatographieschritt auf Basis hydrophober Interaktionen erfolgen.

US 6,008,036 beschreibt eine Methode zur Virusaufreinigung mit Hilfe von zwei Ionenaustauschern in zwei Schritten, wobei für den zweiten Schritt eine anionenaustauschende Chromatographiematrix verwendet wird, wenn im ersten Schritt eine kationenaustauschende Matrix verwendet wird, und umgekehrt.

EP 1 878 791 A1 beschreibt die Aufreinigung von Influenza-Viren mit Hilfe verschiedener Anionenaustauscher, unter anderem mit Gelen, Monolithen und Membranadsorbem. Die beschriebene Methode liefert für Influenza-Viren nur geringe Ausbeuten im Bereich von höchstens 50 %.

B. Kalbfuss et al. offenbaren in "Journal of Membrane Science", 299 (2007), 251-260, die Aufreinigung von Influenza-A-Viren aus Zellkulturen mit Hilfe von Anionenaustausch-Membranen der Typen Sartobind^{®} D MA75 bzw. Sartobind^{®} Q. Das Virus wird an diesem starken bzw. schwachen Anionenaustauscher reversibel gebunden und anschließend eluiert. Die Autoren schlagen vor, zur Erhöhung der Selektivität der jeweiligen Membran für Viren dem Anionenaustausch-Schritt einen Vorbehandlungsschritt vorzuschalten, in dem die aus Nukleinsäuren bestehenden Begleitkontaminanten von den Influenza-Viren abgetrennt werden.

B. Kalbfuss (Dissertation Universität Magdeburg 2009 "Downstream Processing of Influenza Whole-Virions for Vaccine Production") berichtet über die Bindung von Influenza-Viren an einen Sartobind^{®} Q-Membranadsorber, welcher ein starker Anionenaustauscher ist, im Vergleich zur Bindung dieser Viren an einen Sartobind^{®} D-Membranadsorber, welcher ein schwacher Anionenaustauscher ist. Die Virusausbeuten bei Verwendung des Sartobind^{®} Q-Membranadsorbers sind mit 86 % höher als die Virusausbeuten, die bei Verwendung des Sartobind^{®} D-Membranadsorbers (38 %) erzielt werden. Der niedrige isoelektrische Punkt der Begleitkontaminanten (DNS) erlaubt deren starke Adsorption. Über die Einstellung von pH und Ionenstärke kann DNS adsorbiert werden, wobei die Viren durch den Anionenaustauscher durchgeleitet werden, ohne adsorbiert zu werden. Bei Versuchen, DNS als Kontaminante von Influenza-Viren zu trennen, wurde festgestellt, daß es sehr hoher Salzkonzentrationen bedarf, um die Virusadsorption zu unterdrücken. Bei einer Konzentration von 0,15 M NaCl adsorbiert das Virus vollständig am Membranadsorber, bei einer Konzentration von 0,7 M NaCl wurden 10 % des Virus an Sepharose^{®} Q XL adsorbiert. Die hohe Salzkonzentration führt jedoch zum Durchbruch von DNS.

Die im Stand der Technik bekannten, konvektiv permeablen chromatographischen Materialien, wie Membranadsorber (z. B. Produktfamilie Sartobind^{®} der Fa. Sartorius Stedim Biotech GmbH) oder Monolithen der Fa. BIA Separations wurden ursprünglich für die Proteinaufreinigung entwickelt und optimiert, wobei hohe Bindungskapazitäten für Proteine angestrebt wurden. Bei der Aufreinigung von Viren sollen Proteine (z. B. Wirtszellproteine oder Endotoxine) oder Nukleinsäuren als Kontaminanten vom Virus als Zielprodukt entfernt werden. Bei Verwendung der vorgenannten, im Stand der Technik bekannten Verfahren unter Einsatz von Ionenaustauschern werden neben den Viren auch die vorgenannten Kontaminanten adsorbiert, was zu niedrigen Reinheitsgraden und niedrigen Virusausbeuten nach der Desorption des Virus von dem Membranadsorber führt.

T. Vicente et al. beschreiben in "Gene Therapy" 2009, 1-10, ein dreistufiges Verfahren zur Reinigung von Bakuloviren, umfassend einen einleitenden Schritt einer Tiefenfiltration, einen zweiten Schritt einer Ultra- bzw. Diafiltration und einen abschließenden Schritt einer Reinigung von Bakuloviren durch reversible Bindung an einen Anionenaustauscher-Membranadsorber des Typs Sartobind^{®} D MA15.

Der vorliegenden Erfindung liegt die Aufgabe zugrunde, ein Verfahren bereitzustellen, welches die vorgenannten Nachteile des Standes der Technik (niedriger Reinheitsgrad der Viren aufgrund des Vorhandenseins von Kontaminanten sowie niedrige Ausbeute an Viren) überwindet und welches es erlaubt, Viren aus biotechnologischen Fluiden in hoher Ausbeute und hoher Reinheit schnell und kostensparend bereitzustellen.

Diese Aufgabe wird durch das Verfahren nach Anspruch 1 und durch die in den abhängigen Ansprüchen 2 bis 12 charakterisierten Ausführungsformen gelöst.

Die vorliegende Erfindung stellt ein Verfahren zur Abtrennung von Viren aus einem Kontaminanten enthaltenden flüssigen Medium bereit, welches die folgenden Schritte umfaßt:
A) Kontaktieren des flüssigen Mediums mit einem ersten Adsorbens, welches kationische Gruppen enthält,
B) Abtrennen des flüssigen Mediums von dem ersten Adsorbens,
C) Desorbieren der Viren von dem ersten Adsorbens,
D) Kontaktieren eines zweiten Adsorbens, welches kationische Gruppen enthält, mit einem flüssigen Medium, welches die Viren aus Schritt C) und multivalente Anionen umfaßt, und
E) Abtrennen des die Viren enthaltenden, flüssigen Mediums aus Schritt D) von dem zweiten Adsorbens.

Der Begriff "Virus" gemäß der vorliegenden Erfindung unterliegt keiner speziellen Beschränkung und betrifft alle Viren, die durch biotechnologische Verfahren, z. B. durch Zelllyse von Zellkulturen, zugänglich sind. Er schließt beispielsweise Wildtyp-, Mutantenund rekombinante Viren, adenovirale und retrovirale Vektoren für die Expression von heterologen Nukleinsäuresequenzen, Bakuloviren, Bakteriophagen, Virenbestandteile und virenartige Partikel ein.

Der Begriff "Kontaminanten" gemäß der vorliegenden Erfindung betrifft alle Kontaminanten, die als (unerwünschte) Begleitkomponenten in Medien, z. B. nach einer Zelllyse einer Zellkultur, neben den abzutrennenden Viren vorliegen können, wie z. B. Wirtszellproteine, Endotoxine, Nukleinsäuren (DNS, RNS) und Zellbestandteile.

Erfindungsgemäß wird in Schritt D) das zweite Adsorbens mit einem flüssigen Medium kontaktiert, welches die Viren aus Schritt C) und multivalente Anionen umfaßt, um die Kontaminanten durch Adsorption an das zweite Adsorbens von den Viren abzutrennen.

Überraschenderweise wurde gefunden, dass die Effizienz der in Schritt A) und D) verwendeten Anionenaustauscher bezüglich der Entfernung von Kontaminanten, wie DNS, Wirtszellproteinen oder Endotoxinen, aus virushaltigen Lösungen signifikant gesteigert werden kann, wenn dem im Schritt D) verwendeten Medium, d. h. dem virushaltigen Puffer, welcher nach Schritt C) resultiert, multivalente Ionen zugesetzt werden. Die Effizienzsteigerung durch den Zusatz der multivalenten Ionen in Schritt D) manifestiert sich in verkürzten Prozeßzeiten, höheren Virusausbeuten und höherer Virusreinheit. Durch die erfindungsgemäße Kombination von zwei Anionenaustauschern, von denen der erste im "Bind-and-Elute"-Modus und der zweite im "Flow-through"-Modus betrieben werden, läßt sich ein Aufreinigungsprozeß für Viren bereitstellen, der gegenüber den aus dem Stand der Technik bekannten Verfahren die vorgenannten Vorteile aufweist.

Unter "multivalenten Anionen" im Sinne der vorliegenden Erfindung sollen alle Anionen verstanden werden, die mindestens zwei ionogene Ladungen aufweisen. Als Quelle für derartige Anionen können alle geeigneten, in Wasser oder wäßrigen Lösungen löslichen anorganischen oder organischen Salze dienen.

Gemäß der vorliegenden Erfindung sind das "erste Adsorbens" und das "zweite Adsorbens" ausgewählt aus der Gruppe der Chomatographiematrizen umfassend Adsorptionsmembranen, Gele und Monolithe.

Bei einer bevorzugten Ausführungsform werden für das erste und das zweite Adsorbens unterschiedliche Chromatographiematrizen verwendet.

Bei einer alternativen Ausführungsform werden für das erste und das zweite Adsorbens gleiche Chromatographiematrizen verwendet.

Unter den vorgenannten Chromatographiematrizen sind besonders Adsorptionsmembranen bevorzugt. Erfindungsgemäß werden unter Adsorptionsmembranen flächige Adsorbentien mit von der einen Seite zur anderen Seite durchgehenden Poren verstanden, deren Oberfläche durch alle bekannten, positiv geladenen, natürlichen oder synthetischen Liganden funktionalisiert ist. Die für das erfindungsgemäße Verfahren eingesetzten Adsorptionsmembranen können in Modulen, deren Bauformen den in der Membranfiltration üblichen Formen entsprechen (z.B. Wickelmodul, Stapelmodul etc.), vorliegen. Besonders bevorzugt sind konvektiv permeable Adsorptionsmembranen, welche im Unterschied zu partikulären Adsorbentien den Vorteil haben, daß durch Anlegung einer hydraulischen Druckdifferenz zwischen den beiden Seiten ihrer Fläche eine Durchströmung mit einem Medium erzwungen werden kann, wodurch anstelle eines rein diffusen Transports der Adsorbenden in Richtung eines Konzentrationsgradienten ins Innere der Adsorptionsmembran ein konvektiver Transport erreicht wird, der bei hohem Durchfluß sehr viel rascher erfolgen kann als ein diffusiver Stofftransport per se. Dadurch kann ein den partikulären Adsorbentien inhärenter Nachteil, der als "Diffusionslimitierung" bezeichnet wird, vermieden werden, der darin besteht, dass mit zunehmender Partikelgröße des Adsorbens und zunehmender Molmasse des Adsorbenden die erforderliche Zeit zur Einstellung des Adsorptionsgleichgewichts erheblich zunimmt, was sich in einer Verschlechterung der Kinetik auswirkt. Weiterhin besteht der Vorteil darin, dass die Poren zugänglich für große Moleküle und Partikel, wie z.B. Viren, sind. So können Viren - anders als bei partikulären Chromatographiegelen - die Liganden an der gesamten inneren und äußeren Oberfläche der Adsorptionsmembran erreichen.

Bevorzugt sind Ausführungsformen, bei welcher die Adsorptionsmembran eine mikroporöse Membran, bestehend aus einem Cellulosederivat, Polyamid, Poly(ether)sulfon, Polyvinylidendifluorid, Polyacrylnitril, Polyvinylchlorid, Polypropen, Polyethen, Polytetrafluorethen, deren Copolymeren oder deren Gemischen, ist.

Erfindungsgemäß weisen das erste und das zweite Adsorbens kationische Gruppen auf, die aus der Gruppe der primären, sekundären, tertiären oder quaternären Ammoniumgruppen oder Kombinationen davon ausgewählt sind. Besonders bevorzugt sind tertiäre Ammoniumgruppen ausgewählt aus der Gruppe der Trialkyl- und Triarylammoniumgruppen sowie primäre Ammoniumgruppen ausgewählt aus der Gruppe der polymeren Aminoverbindungen mit linearen und/oder verzweigten und/oder cyclischen Strukturen. Bei den polymeren Aminoverbindungen sind am meisten Liganden ausgewählt aus der Gruppe von Polyallylamin, Polyethylenimin oder Polyvinylamin bevorzugt. Als Beispiel für ein mit einem Polyallylaminliganden funktionalisiertes Adsorbens wird die in WO 2009/127285 A1 genannte Adsorptionsmembran des Beispiels 21 genannt.

Das erste und zweite Adsorbens können dabei jeweils gleiche kationische Gruppen oder jeweils verschiedene kationische Gruppen enthalten.

Gemäß einer bevorzugten Ausführungsform der vorliegenden Erfindung sind die multivalenten Anionen ausgewählt aus der Gruppe umfassend Orthophosphat, Monohydrogendiphosphat, Citrat, Carbonat, Nitrid, Oxid, Sulfid, Sulfit und Sulfat sowie Kombinationen davon besonders bevorzugt.

Insbesondere wurde festgestellt, dass unter Zugabe von dreiwertigen Anionen, wie Orthophosphat oder Citrat, die Phagenbindung an das zweite Adsorbens stark reduziert wird, während die Bindung von unerwünschten Kontaminanten (wie z.B. DNS oder Endotoxinen) an das zweite Adsorbens kaum beeinflusst wird. Dieser überraschende Befund wird in dem erfindungsgemäßen Verfahren genutzt, um die vorgenannten Kontaminanten aus virushaltigen Lösungen mittels des zweiten Adsorbens in Schritt D) gemäß einer "negativen Adsorption" ("Flow-Through"-Schritt) zu entfernen. Durch Zugabe dieser dreiwertigen Anionen kann die Selektivität der in dem erfindungsgemäßen Verfahren eingesetzten Adsorbentien zugunsten höherer Virusreinheitsgrade erhöht werden.

Am meisten bevorzugt unter den vorgenannten multivalenten Anionen ist das Orthophosphat-Anion mit drei negativen Ladungen. Bei Verwendung dieses Anions sind besonders hohe Virusausbeuten und Virusreinheitsgrade erzielbar (Vgl. Beispiel).

Bei einer weiteren bevorzugten Ausführungsform der vorliegenden Erfindung beträgt die Ladungsdichte der kationischen Gruppen auf dem ersten und/oder zweiten Adsorbens weniger als 120 µmol/ml, bevorzugt weniger als 100 µmol/ml, besonders bevorzugt weniger als 80 µmol/ml und am meisten bevorzugt weniger als 50 µmol/ml.

Bei einer besonders bevorzugten Ausführungsform beträgt die Ladungsdichte der kationischen Gruppen auf dem ersten Adsorbens weniger als 120 µmol/ml, bevorzugt weniger als 100 µmol/ml, besonders bevorzugt weniger als 80 µmol/ml und am meisten bevorzugt weniger als 50 µmol/ml.

Die zuletzt genannte Ausführungsform des erfindungsgemäßen Verfahrens, wobei das erste Adsorbens, welches in den Schritten A) bis C) erfindungsgemäß im "Bind-and-Elute"-Modus verwendet wird, die vorgenannte Limitierung der Ladungsdichte der kationischen Gruppen aufweist, erlaubt in Synergie mit der Verwendung von multivalenten Anionen zur Durchführung des Schritts D) im "Flow-Through"-Modus, Viren in besonders hoher Ausbeute und Reinheit von begleitenden Kontaminanten aus der Zelllyse einer Zellkultur abzutrennen.

R. Faber et al. haben in "Journal of Chromatography A" 1216 (2009), 941-947, gezeigt, daß Moleküle, die ungefähr 100 nm groß sind, nicht in die aufgepfropfte Gelschicht von Adsorptionsmembranen eindringen können. Dadurch können Partikel solcher Größenordnung nur an der äußeren Oberfläche der Gelschicht adsorbiert werden. Die gepfropfte Gelschicht ist aber für Proteinmoleküle, die eine Größenordnung kleiner sind als Viren, d. h. ca. 10 nm groß sind, zugänglich, wodurch die Proteine in großen Mengen in der dreidimensionalen Gelschicht adsorbiert werden können.

Die vorgenannte Ausführungsform des erfindungsgemäßen Verfahrens mit limitierter Ladungsdichte auf dem ersten Adsorbens zeigt in überraschender Weise als Weiterentwicklung der Befunde von R. Faber et al., daß durch Ladungsdichten unter 120 µmol/ml die Bindung von großen Molekülen, wie abzutrennenden Viren, an das erste Adsorbens im "Bind-and-elute"-Modus nicht beeinflußt wird, dagegen die Bindung der Kontaminanten (Wirtszellproteine, DNS-Fragmente) an das erste Adsorbens in gewünschter Weise weiter reduziert wird und somit der Reinheitsgrad der nach dem erfindungsgemäßen Verfahren gewonnenen Viren erhöht werden kann.

Bei einer weiteren Ausführungsform des erfindungsgemäßen Verfahrens werden nach Schritt C) und vor Schritt D) intermediäre Behandlungsschritte durchgeführt. Bei den intermediären Behandlungsschritten handelt es sich bevorzugt um Tangentialfluß-, Ultra- oder Diafiltrationsschritte, Verdünnungsschritte, Zentrifugationsschritte, Fällungsreaktionen, Chromatographieschritte, Enzymbehandlungsschritte (z. B. mit Nukleasen) oder Kombinationen davon.

Bei einer alternativen Ausführungsform des erfindungsgemäßen Verfahrens kann die Abfolge der Schritte nach Anspruch 1 dahingehend verändert werden, daß die Schritte D) und E) vor den Schritten A) bis C) ablaufen. Die Kontaminanten werden hierbei zunächst durch das erste Adsorbens durch "negative Adsorption" im "Flow-Through"-Modus in Anwesenheit multivalenter Anionen aus dem Viren enthaltenden Medium entfernt, wobei das resultierende Permeat nachfolgend mit dem zweiten Adsorbens zur Adsorption der Viren kontaktiert wird und die Viren nachfolgend von dem zweiten Adsorbens desorbiert werden.

Die vorliegende Erfindung und weitere sich daraus ergebende Vorteile werden unter Bezugnahme auf die in dem Beispiel beschriebenen Ausführungsformen näher erläutert, ohne den Schutzbereich des Anspruchsbegehrens auf diese Ausführungsformen einzuschränken.

### Beispiel:

### Aufreinigung von Bakteriophagen ΦX174 als Modellviren

Als Modellvirus wird der Bacteriophage ΦX174 mit einem Durchmesser von ca. 30 nm und einem isoelektrischen Punkt pI von 6,4 - 6,7 verwendet. Zur Entfernung der Kontaminanten (DNS) werden als erstes und zweites Adsorbens, welche kationische Gruppen enthalten, Anionenaustauscher der Fa. Sartorius Stedim Biotech GmbH verwendet (Membran A: Sartobind^{®} Q, Membran B: mit Polyallylamin modifizierte Cellulosemembran wie im Beispiel 21 von WO 2009/127285 A1 beschrieben). Dabei wurden zwei verschiedene Kombinationen von Anionenaustauschern verwendet:
Kombination 1): Erstes Adsorbens: Membran A, zweites Adsorbens: Membran A sowie
Kombination 2): Erstes Adsorbens: Membran A, zweites Adsorbens: Membran B.

Während der Schritte A) und B) des erfindungsgemäßen Verfahrens werden die Phagen auf der Membran A adsorbiert und anschließend in Schritt C) desorbiert. Dies entspricht einem "Bind-and-Elute"-Schritt. Die darauffolgenden Schritte D) und E) werden wahlweise mit Membran A und B als "negative Adsorption" im "Flow-through"-Modus durchgeführt.

### Kultivierung der Phagen

Die Phagen werden in einer Kultivierung von Escherichia coli C (ATCC 13706) produziert. Nach der Anzucht in einem Einweg-Bioreaktor (50 1 Biostat^{®} CultiBag RM, Sartorius Stedim Biotech GmbH, Göttingen, Deutschland) und Animpfen der Bakterienkultur wird 4 h lang kultiviert. Anschließend wird die Kultivierung beendet. Die Phagen werden über eine Filtrationskaskade (Sartopure^{®} PP2 MidiCap 8µm - 5µm und Sartopore^{®} 2XLG 0,8 - 0,2 µm, Sartorius Stedim Biotech GmbH) partikelfrei und steril filtriert. Durch Crossflow-Filtration (Sartocon^{®} Slice Casette 30 kDa, Sartorius Stedim Biotech GmbH) wird das Endvolumen auf 600 ml reduziert. Diese Lösung wird mit einer 30 %igen wäßrigen Polyethylenglykol-Lösung gemischt und anschließend 90 min bei 8430 Umdrehungen/min zentrifugiert (Sorvall RC-6 Zentrifuge, Thermo Fisher Scientific, Bonn, Deutschland). Die Pellets werden mit 25 ml 25 mM Tris-Pufferlösung (Tris-hydroxymethyl-aminomethan, pH=8,0) resuspendiert, aliquotiert und bei -70 °C gelagert. Diese Stammlösung ist das Ausgangsmaterial für das hier erläuterte Beispiel und wird nach Bedarf entsprechend verdünnt.

Der Bestimmung des Phagentiters erfolgt durch Titration. Dazu werden die Proben entsprechend verdünnt und mit dem Wirtsorganismus (Escherichia coli C) zusammengegeben. Nach der Inkubationszeit von 10 min werden die Proben ausplattiert und über Nacht bei 37 °C bebrütet. Am nächsten Tag werden die Plaques ausgezählt und der Titer berechnet. Die Bestimmung der DNS-Konzentration für den zweiten Prozeßschritt im Beispiel erfolgt nach Herstellerangaben mit "PicoGreen" (Quant-iT^{®} PicoGreen dsDNA Reagenz P7581, Life Technologies, Carlsbad, USA) unter Verwendung eines Plattenreaders (Tecan Safire, Tecan Trading AG, Schweiz). Die Fluoreszenz wurde bei einer Anregung von 480 nm und die Absorption bei 520 nm gemessen (Greiner Fluotrac^{®} 200, Greiner Bio-One GmbH, Frickenhausen, Deutschland). Für jeden Versuch werden die Membranen A bzw. B dreilagig in einen Halter eingebaut. Die Gesamtfläche der Membranen betrug 15 cm².

### Adsorption und Desorption der Phagen / Schritte A) bis C) des erfindungsgemäßen Verfahrens

Im ersten Schritt wird die Stammlösung 1:100 mit 20 mM Tris-Puffer (pH=8,1) verdünnt. Das phagenhaltige, flüssige Medium mit dem Phagentiter von 6x10⁷ PFU/ml wird mit der Membran A in Kontakt gebracht, wobei die Phagen an der Membran adsorbieren (PFU = "Plaque forming units"). Die Filtration erfolgt bei allen Schritten bei einer Flußrate von 20 ml/min mit einer Schlauchpumpe. Vor der Filtration wird die Membran A mit 10 ml Puffer konditioniert (1 M Natriumchlorid, 20 mM Tris [Tris-hydroxymethyl-aminomethan], pH=8,1) und mit 10 ml Bindungspuffer gewaschen (20 mM Tris, pH 8,1). Anschließend werden 800 ml der Phagenlösung im Bindungspuffer filtriert (Beladung, Kontaktieren des flüssigen Mediums mit Adsorbens). Nach der Beladung wird die Membran A erneut mit 30 ml Bindungspuffer gewaschen. Weiterhin wird die Membran A mit einem Elutionsmedium (250 ml 150 mM Natriumchlorid in Bindungspuffer) beaufschlagt. In Tabelle 1 sind die Phagenkonzentrationen in PFU/ml (Plaque bildende Einheiten pro ml Lösung) in einzelnen Fraktionen angegeben. Die Wiederfindungsrate beträgt nach der Elution der Phagen vom ersten Adsorbens (= Membran A) aufgrund der analytischen Schwankungen bei der Meßmethode mehr als 100 %, hier 119 %, d. h. nach Schritt C) des erfindungsgemäßen Verfahrens können die Phagen quantitativ vom ersten Adsorbens eluiert werden, d. h. ihre Ausbeute beträgt praktisch 100 %

**Tabelle 1: Phagenkonzentration und Wiederfindungsrate nach Schritt C)**

| | Phagenkonzentration | Wiederfindung |
|---|---|---|
| | PFU/ml | % |
| Beladung | 6,18 * 10⁷ | 100 |
| Durchlauf | 7,76 * 10³ | 0,013 |
| Waschen | 9,33 * 10² | 0,001 |
| Elution | 2,34 * 10⁸ | 119 |

### "Negative Adsorption" der Kontaminanten im "Flow-through"-Modus am zweiten Adsorbens (Schritte D) und E) des erfindungsgemäßen Verfahrens

Bereits in dem die Phagen enthaltenden Eluat, welches nach Schritt C) des erfindungsgemäßen Verfahrens erhalten wird, kann DNS mittels der "PicoGreen"-Methode nachgewiesen werden (ca. 200 ng/ml). Dabei kann es sich um freie Bruchstücke der Phagen-DNS oder Wirtszell-DNS aus der Kultivierung von Escherichia Coli handeln. Durch die Verdünnung der konzentrierten Elutionslösung (Verdünnung der Phagenkonzentration von 2,34 * 10⁸ PFU/ml, vgl. Tabelle 1, auf 1,00 _{*} 10⁷ PFU/ml) nach Durchführung von Schritt C) des erfindungsgemäßen Verfahrens wird die DNS-Konzentration verringert, wodurch man sich der DNS-Bestimmungsgrenze der "PicoGreen"-Methode nähert.

Um nachzuweisen, daß das erfindungsgemäße Verfahren im "Flow-Through"-Modus der Schritte D) und E) die effiziente Abtrennung der Viren von den Kontaminanten auch aus Stammlösungen ermöglicht, die eine hohe Belastung mit Kontaminanten aus der vorhergehenden Zelllyse enthalten, wird dem nach Schritt C) gewonnenen Eluat als Modellkontaminante Desoxyribonukleinsäure "Salmon Sperm DNA", Lot 8087, der Biomol GmbH, Hamburg, zugesetzt. Hierbei wird eine Konzentration an 200 ng/ml dieser Modell-DNS eingestellt. Damit entspricht die Konzentration an DNS in der für die Schritte D) und E) eingesetzten phagenhaltigen Lösung der Konzentration an DNS in der nach Schritt C) erhaltenen Elutionslösung.

Die nachfolgenden Ergebnisse beweisen, daß das erfindungsgemäße Verfahren erlaubt, auch dann Phagen in hoher Ausbeute und Reinheit aus einer biotechnologischen Stammlösung von Kontaminanten abzutrennen, wenn das Eluat trotz der Schritte A) bis C), bei welchen bereits ein Teil der Kontaminanten durch das erste Adsorbens entfernt wird, mit einer hohen Kontaminantenbelastung dem zweiten Adsorbens zugeführt wird.

Die nachfolgenden Schritte D) und E) erfolgen erfindungsgemäß als "negative Adsorption" im "Flow-through"-Modus. Die beiden Hauptkomponenten im Eluat aus Schritt C) (Phage als Zielsubstrat, und "Salmon Sperm"-DNS als Kontaminante) haben eine negative Nettoladung im neutralen pH-Bereich und binden an die positiv geladenen Membranen A (Kombination 1)) oder B (Kombination 2), vgl. S. 13), wobei die Abtrennung der Phagen von den Kontaminanten durch die Zugabe von Natriumorthophosphat als Quelle für ein multivalentes Anion ermöglicht wird.

Für die Schritte D) und E) werden im folgenden zwei Varianten (Kombination 1) und Kombination 2), vgl. S. 13) beschrieben:

### Membran A (Kombination 1))

Für die Membran A wurde ein Teil der Elutionslösung nach Schritt C) entsprechend verdünnt, um eine NaCl-Konzentration von 6 mM in 20 mM/L Tris-Puffer bei pH 7,5 zu erhalten. Der Phagentiter beträgt danach 1x10⁷ PFU/mL.

### Membrane B (Kombination 2))

Für die Membran B wurde der verbleibende Teil der Elutionslösung nach Schritt C) ebenfalls auf einen Phagentiter von 1x10⁷ PFU/mL eingestellt, wobei die NaCl-Konzentration von 150 mM (in 20 mM Tris-Puffer bei pH 7,5) konstant bleibt. Der Phagentiter beträgt danach 1x10⁷ PFU/mL.

Es werden jeweils 4 verschiedene Pufferzusammensetzungen getestet. Charakterisiert wird das Verhalten des zweiten Adsorbens bei der Zugabe von 0, 2, 10 und 30 mM Natriumorthophosphat zum phagenhaltigen Medium, welches das Eluat aus Schritt C) umfaßt (vgl. Tabelle 2).

**Tabelle 2:**

| Pufferzusammensetzung | Membran A | Membran B |
|---|---|---|
| Monovalentes Anion Chlorid [mM] | 6 | 150 |
| Multivalentes Anion Orthophosphat [mM] | 0-30 | 0-30 |
| TRIS [mM] | 20 | 20 |

Nach dem Waschschritt mit 50 mL Bindungspuffer wurden 150 ml des Gemisches aus Phagen- und DNS-Lösung bei einer Flußrate von 10 ml/min filtriert. Durch die Analyse der Konzentrationen der einzelnen Komponenten in den Ausgangslösungen und Durchlauffraktionen kann sowohl für die DNS als auch für die Phagen die Rückhaltung charakterisiert werden (vgl. Tabelle 3).

**Tabelle 3: Prozentualer Anteil in % der Ausgangskonzentration an Phagen und Kontaminanten (DNS) im Durchlauf nach Schritt E) des erfindungsgemäßen Verfahrens**

| Orthophosphatkonzentration [mM] | Kombinat. 1) Membran A Phage | Kombinat. 1) Membran A DNS | Kombinat. 2) Membran B Phage | Kombinat. 2) Membran B DNS |
|---|---|---|---|---|
| 0 | 0,001 | < 1 | < 0,00001 | < 1 |
| 2 | 0,001 | < 1 | 0,0001 | < 1 |
| 10 | 0,01 | < 1 | 0,001 | < 1 |
| **30** | **59 - 78** | **<** 1 | **75 - 83** | **< 1** |

Für alle getesteten Pufferkonfigurationen beträgt die gemessene Konzentration an DNS in den Proben des Permeatstroms nach Schritt E) weniger als 1 % der DNS-Konzentration der Ausgangslösung. Für die betrachteten Pufferzusammensetzungen bleibt die Bindung der DNS an die Ionentauscher-Membranen A und B annähernd konstant hoch. Die nichtbindenden Bedingungen für die Phagen, unter welchen die Phagen in hoher Ausbeute gewonnen werden können, werden bei Natriumorthophosphatkonzentration ab 30 mM erreicht.

## Patentansprüche

1. Verfahren zur Abtrennung von Viren aus einem Kontaminanten enthaltenden flüssigen Medium, umfassend die folgenden Schritte:
A) Kontaktieren des flüssigen Mediums mit einem ersten Adsorbens,
welches kationische Gruppen enthält,
B) Abtrennen des flüssigen Mediums von dem ersten Adsorbens,
C) Desorbieren der Viren von dem ersten Adsorbens,
D) Kontaktieren eines zweiten Adsorbens, welches kationische Gruppen enthält, mit einem flüssigen Medium, welches die Viren aus Schritt C) und multivalente Anionen umfaßt, und
E) Abtrennen des die Viren enthaltenden, flüssigen Mediums aus Schritt D) von dem zweiten Adsorbens.

2. Verfahren nach Anspruch 1, wobei das erste Adsorbens und
das zweite Adsorbens ausgewählt sind aus der Gruppe der Chomatographiematrizen umfassend Adsorptionsmembranen, Gele und Monolithe.

3. Verfahren nach Anspruch 2, wobei für das erste und das zweite Adsorbens unterschiedliche Chromatographiematrizen verwendet werden.

4. Verfahren nach Anspruch 2, wobei für das erste und das zweite Adsorbens gleiche Chromatographiematrizen verwendet werden.

5. Verfahren nach einem der vorhergehenden Ansprüche 2 bis 4, wobei die Adsorptionsmembran eine mikroporöse Membran, bestehend aus einem Cellulosederivat, Polyamid, Poly(ether)sulfon, Polyvinylidendifluorid, Polyacrylnitril, Polyvinylchlorid, Polypropen, Polyethen, Polytetrafluorethen, deren Copolymeren oder deren Gemischen, ist.

6. Verfahren nach einem der vorhergehenden Ansprüche, wobei die multivalenten Anionen ausgewählt sind aus der Gruppe umfassend Orthophosphat, Monohydrogendiphosphat, Citrat, Carbonat, Nitrid, Oxid, Sulfid, Sulfit und Sulfat oder Kombinationen davon.

7. Verfahren nach einem der vorhergehenden Ansprüche, wobei die Ladungsdichte der kationischen Gruppen auf dem ersten und/oder zweiten Adsorbens weniger als 120 µmol/ml, bevorzugt weniger als 100 µmol/ml, besonders bevorzugt weniger als 80 µmol/ml und am meisten bevorzugt weniger als 50 µmol/ml beträgt.

8. Verfahren nach einem der vorhergehenden Ansprüche, wobei nach Schritt C) und vor Schritt D) intermediäre Behandlungsschritte durchgeführt werden.

9. Verfahren nach Anspruch 8, wobei es sich bei den intermediären Behandlungsschritten um Tangentialfluß-, Ultra- oder Diafiltrationsschritte, Verdünnungsschritte, Zentrifugationsschritte, Fällungsreaktionen, Chromatographieschritte, Enzymbehandlungsschritte oder Kombinationen davon handelt.

10. Verfahren nach einem der vorhergehenden Ansprüche, wobei die kationischen Gruppen des ersten und zweiten Adsorbens aus der Gruppe umfassend primäre, sekundäre, tertiäre oder quaternäre Ammoniumgruppen oder Kombinationen davon ausgewählt sind.

11. Verfahren nach Anspruch 10, wobei das erste Adsorbens als kationische Gruppen Trimethylammoniumgruppen und das zweite Adsorbens als kationische Gruppen Trimethylammoniumgruppen oder Ammoniumgruppen basierend auf PolyallylaminLiganden aufweisen.

12. Verfahren nach einem der vorhergehenden Ansprüche, wobei die Kontaminanten ausgewählt sind aus der Gruppe umfassend Zellkulturbestandteile, Nukleinsäuren, Endotoxine oder Wirtszellproteine.

## Claims

1. A method for the purification of viruses from liquids containing contaminants, comprising the following steps:
A) contacting the liquid with a first adsorbent containing cationic groups,
B) separating the liquid from the first adsorbent,
C) desorbing the viruses from the first adsorbent,
D) contacting a second adsorbent containing cationic groups with a liquid comprising the viruses from step C) and multivalent anions, and
E) purifying the virus-containing liquid from step D) from the second adsorbent.

2. The method according to Claim 1, wherein the first adsorbent and the second adsorbent are selected from the group of chromatography matrices comprising adsorption membranes, gels and monoliths.

3. The method according to Claim 2, wherein different chromatography matrices are used for the first and the second adsorbent.

4. The method according to Claim 2, wherein identical chromatography matrices are used for the first and the second adsorbent.

5. The method according to any of the preceding Claims 2 to 4, wherein the adsorption membrane is a microporous membrane consisting of a cellulose derivative, polyamide, poly(ether)sulfone, polyvinylidene difluoride, polyacrylonitrile, polyvinyl chloride, polypropene, polyethene, polytetrafluoroethene, copolymers thereof or mixtures thereof.

6. The method according to any one of the preceding claims, wherein the multivalent anions are selected from the group comprising orthophosphate, monohydrogendiphosphate, citrate, carbonate, nitride, oxide, sulfide, sulfite and sulfate or combinations thereof.

7. The method according to any of the preceding claims, wherein the charge density of the cationic groups on the first and/or the second adsorbent is less than 120 µmol/mL, preferably less than 100 µmol/mL, particularly preferably less than 80 µmol/mL and most preferably less than 50 µmol/mL.

8. The method according to any of the preceding claims, wherein intermediate treatment steps are carried out after step C) and before step D).

9. The method according to Claim 8, wherein the intermediate treatment steps are tangential flow filtration, ultrafiltration or diafiltration steps, dilution steps, centrifugation steps, precipitation reactions, chromatography steps, enzyme treatment steps or combinations thereof.

10. The method according to any of the preceding claims, wherein the cationic groups of the first and the second adsorbent are selected from the group comprising primary, secondary, tertiary and quaternary ammonium groups and combinations thereof.

11. The method according to Claim 10, wherein the first adsorbent has, as cationic groups, trimethylammonium groups and the second adsorbent has, as cationic groups, trimethylammonium groups or ammonium groups based on polyallylamine ligands.

12. The method according to any of the preceding claims, wherein the contaminants are selected from the group comprising cell-culture constituents, nucleic acids, endotoxins or host-cell proteins.

## Revendications

1. Un procédé pour purifier des virus de liquides contenant des contaminants, ledit procédé consistant à:
a) mettre le liquide en contact avec un premier adsorbant contenant des groupes cationiques;
b) séparer le liquide du premier adsorbant;
c) désorber les virus du premier adsorbant;
d) mettre un second adsorbant contenant des groupes cationiques en contact avec un liquide comprenant les virus de l'étape (c) et les anions polyvalents; et
e) purifier le liquide contenant les virus de l'étape (d) du second adsorbant.

2. Le procédé selon la revendication 1, le premier et le second adsorbant étant sélectionnés parmi le groupe de matrices chromatographiques comprenant des membranes d'adsorption, des gels et des monolithes.

3. Le procédé selon la revendication 2, des matrices de chromatographie différentes étant utilisées pour le premier et le second adsorbant.

4. Le procédé selon la revendication 2, des matrices de chromatographie identiques étant utilisées pour le premier et le second adsorbant.

5. Le procédé selon une quelconque des précédentes revendications 2 à 4, la membrane d'adsorption étant une membrane microporeuse composée de: un dérivé cellulosique, un polyamide, un polyéther sulfone, un difluorure de polyvinylidène, un polyacrylonitrile, un polychlorure de vinyle, un polypropylène, un polyéthylène, un polytétrafluoréthylène, des copolymères ou mélanges de ceux-ci.

6. Le procédé selon une quelconque des revendications précédentes, les anions polyvalents étant sélectionnés parmi un groupe comprenant l'orthophosphate, le monohydrogénodiphosphate, le citrate, le carbonate, le nitrure, l'oxyde, le sulfure, le sulfite et le sulfate ou les combinaisons de ceux-ci.

7. Le procédé selon une quelconque des revendications précédentes, la densité de charge des groupes cationiques sur le premier et/ou le second adsorbant étant inférieure à 120 µmol/ml, préférablement à 100 µmol/ml, encore plus préférablement à 80 µmol/ml et le plus préférablement à 50 µmol/ml.

8. Le procédé selon une quelconque des revendications précédentes, les étapes de traitement intermédiaire étant réalisées après l'étape (c) et avant l'étape (d).

9. Le procédé selon la revendication 8, le traitement intermédiaire étant réalisé aux étapes de:
filtration à courant tangentiel, ultrafiltration ou diafiltration, dilution, centrifugation,
réactions de précipitation, chromatographie, traitement enzymatique ou par des combinaisons de celles-ci.

10. Procédé selon une quelconque des revendications précédentes, les groupes cationiques du premier et du second adsorbant étant sélectionnés parmi le groupe comprenant les groupes d'ammonium primaires, secondaires, tertiaires et quaternaires et des combinaisons de ceux-ci.

11. Le procédé selon la revendication 10, le premier adsorbant ayant, en tant que groupes cationiques, des groupes de triméthylammonium et le second adsorbant ayant, en tant que groupes cationiques, des groupes de triméthylammonium ou d'ammonium basés sur des ligands de polyallylamine.

12. Le procédé selon une quelconque des revendications précédentes, les contaminants étant sélectionnés parmi un groupe comprenant des composants de culture cellulaire, des acides nucléiques, des endotoxines ou des protéines de cellules hôtes.
